# EUROPEAN PATENT APPLICATION

(11) **EP 4 491 106 A1**
(43) Date of publication of application: **15.01.2025**
(21) Application number: 24188410.5
(22) Date of filing: 12.07.2024
(51) Int. Cl.: A61B 5/00, A61B 5/0507, A61B 5/08, G01S 7/00

(54) **SYSTEM AND METHOD FOR MONITORING FLUID ACCUMULATION IN AN ORGAN**

(30) Priority: 14.07.2023 US 202318222159
(71) Applicant: Robert Bosch GmbH, 70469 Stuttgart (DE)
(72) Inventor: Munir, Sirajum, Pittsburgh, PA, 15229 (US); Wang, Wengpeng, Pittsburgh, PA, 15222 (US); Das, Samarjit, Wexford, PA, 15090 (US); Ghaffarzadegan, Shabnam, Livermore, CA, 94550 (US)
(74) Representative: Isarpatent

(57) **Abstract**

A system includes a first and second transceiver configured to emit and receive a wireless signal, a controller in communication with the first and second transceiver, the controller configured to send instructions to the transceivers to initiate a Fine Time Measurement (FTM) on a wireless channel to a body part associated with a user during a healthy state of the user, store an FTM measurement associated with the healthy state of the user, send instructions to the transceiver to initiate the FTM on the wireless channel during an infected state of the user, store an FTM measurement associated with the infected state of the user, compare the FTM measurement associated with the healthy state of the user with the FTM measurement associated with the infected state of the user, and in response to the comparison exceeding a threshold value, output a notification.

## Description

### TECHNICAL FIELD

The present disclosure relates to utilizing wireless technology, including wireless technology for medical purposes.

### BACKGROUND

COVID-19 mortality may be driven by abnormal alveolar fluid metabolism of the lung. This causes fluid accumulation in the air sacs of the lungs, which is referred to as pulmonary edema. When it happens, it becomes difficult to breathe and it can potentially cause respiratory failure.

### SUMMARY

A first embodiment discloses, a system includes a first and second transceiver configured to emit and receive a wireless signal, a controller in communication with the first and second transceiver, the controller configured to send instructions to the transceivers to initiate a Fine Time Measurement (FTM) on a wireless channel to a body part associated with a user during a healthy state of the user, store an FTM measurement associated with the healthy state of the user, send instructions to the transceiver to initiate the FTM on the wireless channel during an infected state of the user, store an FTM measurement associated with the infected state of the user, compare the FTM measurement associated with the healthy state of the user with the FTM measurement associated with the infected state of the user, and in response to the comparison exceeding a threshold value, output a notification.

A second embodiment discloses, a system includes a first transceiver configured to emit a wireless signal, a second transceiver configured to receive the wireless signal, a controller in communication with the first transceiver and second transceiver. The controller is configured to send instructions to the first transceiver to initiate a Fine Time Measurement (FTM) on a wireless channel to a body part associated with a user during a healthy state of the user, store an FTM measurement associated with the healthy state of the user, send instructions to the first transceiver to initiate the FTM on the wireless channel during an infected state of the user, store an FTM measurement associated with the infected state of the user, compare the FTM measurement associated with the healthy state of the user with the FTM measurement associated with the infected state of the user, and in response to the comparison exceeding a threshold value, output a notification.

A third embodiment discloses, a system that includes a transmitter configured to emit a wireless signal, a receiver configured to receive the wireless signal and extract CSI data associated with the received signal, a controller in communication with the transmitter and the receiver, the controller configured to send instructions to the transmitter to send a first set of packets on a wireless channel to a body part associated with a user during a healthy state of the user; store a healthy CSI measurement associated with the healthy state of the user; send instructions to the transceivers to initiate sending a second set of packets on the wireless channel during an infected state of the user, store an infected CSI measurement associated with the infected state of the user, compare the healthy CSI measurement associated with the healthy state of the user with the infected CSI measurement associated with the infected state of the user, and in response to the comparison exceeding a threshold value, output a notification.

### BRIEF DESCRIPTION OF THE DRAWINGS

FIG. 1 illustrates an embodiment of a drawing of a lung with a healthy air sac versus one with an infected air sac.
FIG. 2 illustrates an example diagram of a transceiver unit.
FIG. 3 illustrates a flow chart of a transmitter and receiver communicating to one another utilizing Wi-Fi fine-time measurement based distance estimation according to an embodiment.
FIG. 4A illustrates a Wi-Fi fine-time measurement to determine fluid accumulation with a dry sponge.
FIG. 4B illustrates a Wi-Fi fine-time measurement to determine fluid accumulation with a wet sponge.
FIG. 5A illustrates a Wi-Fi channel state information to determine fluid accumulation with a dry sponge.
FIG. 5B illustrates a Wi-Fi channel state information to determine fluid accumulation with a wet sponge.

### DETAILED DESCRIPTION

Embodiments of the present disclosure are described herein. It is to be understood, however, that the disclosed embodiments are merely examples and other embodiments can take various and alternative forms. The figures are not necessarily to scale; some features could be exaggerated or minimized to show details of particular components. Therefore, specific structural and functional details disclosed herein are not to be interpreted as limiting, but merely as a representative basis for teaching one skilled in the art to variously employ the embodiments. As those of ordinary skill in the art will understand, various features illustrated and described with reference to any one of the figures can be combined with features illustrated in one or more other figures to produce embodiments that are not explicitly illustrated or described. The combinations of features illustrated provide representative embodiments for typical applications. Various combinations and modifications of the features consistent with the teachings of this disclosure, however, could be desired for particular applications or implementations.

The system described herein proposes to leverage wireless signals to determine fluid accumulation in the lungs. When wireless signals are propagated through a fluid, the fluid attenuates the signal and may affect the amplitude and phase of the wireless signal. To realize this, the system may propose to place a wireless transmitter (TX) and a receiver (RX) across a lung. The idea is to transmit wireless signals using TX and receive the signals at RX under two conditions: (i) when the patient is healthy and (ii) when the patient has respiratory issues. By comparing signal properties when the patient is healthy, the system determines whether there is a fluid build-up in the lungs. The core technology can be useful for determining fluid accumulation in any organ and for other illnesses, e.g., cystic fibrosis, pneumonia, congestive heart failure, kidney failure, or liver cirrhosis

In one embodiment, the idea may to be to transmit wireless signals using transmitter TX and receive the signals at receiver RX under two conditions: (i) when the patient is healthy and (ii) when the patient has respiratory issues. The transceiver (e.g., TX an RX) can be connected to any electronic device (such as a laptop, tablet, phone, etc.) so that it may control the data collection and analysis procedure.

In one embodiment, the transceiver and receiver may be connected to a computer, such as a laptop, or any type of mobile device. In such an embodiment, the transmitter may send Wi-Fi packets that are received by RX. The TX and RX can be swapped, or they may take turns transmitting and receiving. TX and RX can have directional antennas to ensure wireless signals are forced to go through a specific area of the human body. By comparing signal properties when the patient was healthy, the system determines whether there is a fluid build-up in the lungs.

The system described below may include two illustrative embodiments that compare wireless signal properties between a healthy organ and an organ with fluid accumulation.

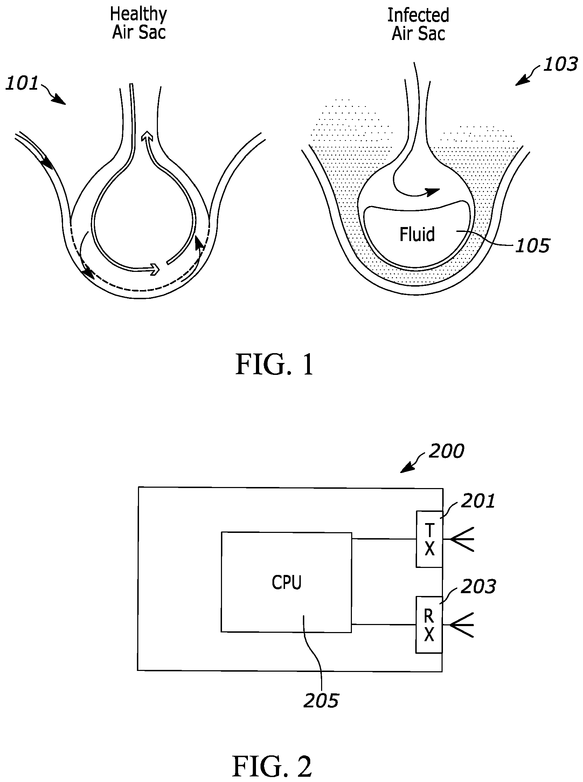

FIG. 1 illustrates an embodiment of a drawing of a lung with a healthy air sac versus one with an infected air sac. As shown, the healthy air sac 101 may allow air to circulate throughout the entire lung. The path of travel for the air may be greater because it follows the entire lung. The flow of air in the infected air sac 103 may be different. Because of fluid 105 that accumulates, the infected air sac 103 may not have the same capacity of the health air sac 101. Thus, the air flow may be a smaller capacity.

FIG. 2 illustrates an example of a transceiver according to one embodiment of the disclosure. The transceiver 200 may include a transmitter 201 (TX) and a receiver 203 (RX). The apparatus may also include a controller or processor 205 that is utilized to communicate with the transmitter 201 and/or receiver 203. In one embodiment, the transmitter 201 and receiver 203 may be part of a unitary transceiver or may be separated.

The communication interface may include a wireless transmitter or transceiver for wirelessly communicating over a communications network. Alternatively or in addition to a wireless transmitter or transceiver, the communication interface may include a port for receiving a cable, such as an Ethernet cable, for communicating over the communications network or a dedicated wired connection. It should be understood that, in some embodiments, the controller or processor 205 may communicate with other devices through one or more intermediary devices, such as routers, gateways, relays, and the like.

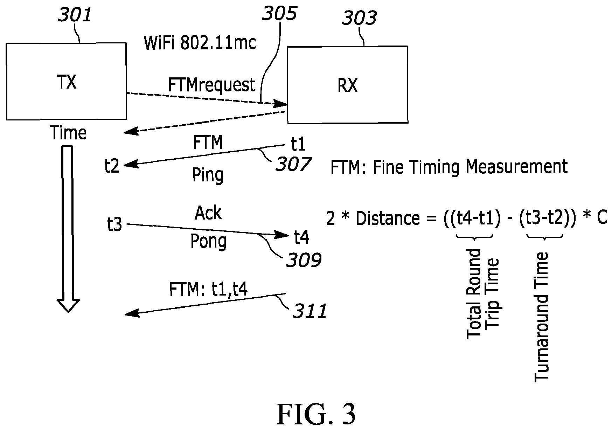

FIG. 3 illustrates a flow chart of a transmitter and receiver communicating to one another utilizing Wi-Fi fine-time measurement based distance estimation according to an embodiment. In such an embodiment, wireless Round Trip Time (RTT) may be leveraged as an effective feature to differentiate a healthy organ from an organ with fluid accumulation. RTT can be estimated by modifying the Wi-Fi firmware, or it can be estimated using Wi-Fi Fine Time Measurement (FTM), which may be a part of the IEEE 802.11mc standard. In order to enable Wi-Fi FTM, both TX and RX need to transmit and receive.

In one embodiment, a transmitter TX initiates an FTM request to a receiver RX on a particular Wi-Fi channel. The channel could be an operating channel in 2.4 GHz, 5 GHz, or another Wi-Fi band. Alternatively, it can perform channel hopping across the entire 2.4 GHz, 5 GHz, or another Wi-Fi band and send an FTM request for each Wi-Fi channel or a set of Wi-Fi channels by channel hopping. Then RX and TX transmit a series of packets to estimate Round Trip Time, which is then translated to the distance between TX and RX. An example of such message exchange and how the RTT estimation is translated to distance is shown in FIG. 3.

The controller may execute an instruction or an algorithm for determining fluid accumulation in an organs. The instructions may include a step 301 that the user sets up the unit by placing transmitter TX and receiver RX across the organ of interest that needs to be monitored for fluid accumulation. At step 303, when the user is healthy, the first set of FTM measurements may be performed using a transmitter and receiver. For example, the system may determine the average value of the distance from the FTM measurements on a particular Wi-Fi channel, which may be referred to as Dₙₒᵣₘₐₗ. These values may be stored in the hardware in any type of memory, including volatile or non-volatile memory. The values may later be used in a comparison. Later, when the user is not feeling well or wants to test for fluid accumulation, the user can collect another round of FTM measurements using a transceiver on a similar setup. In one example, the average value of the distance from the FTM measurements on the same Wi-Fi channel may be defined as Dtest.

If (Dₜₑₛₜ - Dₙₒᵣₘₐₗ) > T, then the system may out a notification or report fluid accumulation in an organ. In such an example, T may be a threshold that indicates that there may be an infection or fluid in a body part of a user. The threshold T may be determined by collecting data across the target population. The threshold may also be adjusted or defined in a look-up table based on attributes of the user. For example, threshold T can be adjusted based on data indicating age, gender, height, weight, and BMI.

The analysis can be done at the transceiver, the transmitter, receiver, or any controller/processor in communication with the system. In another example, the analysis can be conducted on a small computer attached to or communicating with the transceiver and/or components. In one embodiment, the result of the finding (fluid accumulation or not) can be communicated via outputting audible notifications, such as two different beep sounds to differentiate both cases. In another embodiment, the result can be visualized in a small display on an electronic device (e.g. phone/tablet/laptop) through an application or another interface. In another embodiment, instead of using the average value of the data measured, the median value may be used.

In this embodiment, wireless Channel State Information (CSI) is leveraged as an effective feature to differentiate a healthy organ from an organ with fluid accumulation. CSI captures how a signal propagates from the transmitter to the receiver and represents the combined effect of scattering, fading, and power decay with distance.

In one embodiment, TX sends Wi-Fi packets on a particular Wi-Fi channel that the RX is configured to receive the packet in that channel. Upon receiving the packets, RX extracts Wi-Fi Channel State Information associated with each packet. In another embodiment, the position and/or role of TX and RX are swapped.

There may be various embodiments for an algorithm for determining fluid accumulation in an organ. In one example, the user may setup up the unit by placing TX and RX across the organ of interest that needs to be monitored for fluid accumulation. The system may first call for a scan of a user in a health state and store such a condition or measurements. When the user is healthy, the first set of CSI measurements are performed using TX and RX. TX can send N packets. In one embodiment, N can be 20,000. A window of size W is defined to split the N packets into chunks of size W. In one embodiment, W can be 4000. An example of CSI values within a window a under normal condition is shown in Figure 7(a), where the X axis represents different subcarriers, the Y axis represents different packets, and the Z axis represents the amplitude of the CSI values. In another embodiment, instead of using CSI amplitudes, CSI phases are used. This process generates K samples, where K = N/W. Let's represent these samples in a set as Snromal = {S1-normal, S2-normal, ...., SK-normal}. In another embodiment, a sliding window is used to include the same data in multiple contiguous windows. These values are stored in the hardware, where CSI is extracted. In another embodiment, a scan of a healthy user may not be necessary to establish a baseline measurement. For example, in one embodiment, measurements from multiple users may be utilized to estimate a baseline measurement. Such measurements may include weight, BMI, circumference of waist, chest, etc. The system may be able to establish a baseline measurement for all users and then utilize such a baseline for a subsequent analysis.

Later, when the user is not feeling well or wants to test for fluid accumulation, he can collect another round of CSI measurements using TX and RX on a similar setup. Similarly, TX can send N packets. Similarly, a window of size W is defined to split the N packets into chunks of size W. An example of CSI values within a window under an abnormal/unhealthy condition is shown in Figure 7(b), where the X axis represents different subcarriers, the Y axis represents different packets, and the Z axis represents the amplitude of the CSI values. In another embodiment, instead of using CSI amplitudes, CSI phases are used. This process generates K samples, where K = N/W. To represent these samples in a set as Stest = {S1-test, S2-test, ...., SK-test}.

In this step, the difference between Snormal and Stest is computed. This process generates K sets of vecotrs from {S1-normal - S1-test, S2-normal - S2-test, ..., SK-normal - SK-test}. Let's call this set as Sdiff = {Sdiff1, Sdiff2, ..., Sdiff-K}

In this step, Sdiff is fed to a classifier to classify if Stest is significantly different than Snormal, which could potentially mean that there is fluid accumulation in the organ.

The analysis can be done at the TX, RX, or on a small computer attached to them. The result of the finding (fluid accumulation or not) can be communicated via playing two different beep sounds to differentiate both cases, or the result can be visualized in a small display on a phone/tablet/laptop through an app/application.

Before using the classifier, a machine learning model is trained with Sdiff values with a statistically significant population with and without fluid accumulation for each organ of interest by taking into account age, gender, height, weight, BMI, and other bodily features. Then a classifier is used to classify whether there is fluid accumulation or not. For the machine learning model and classification, a Decision Tree (DT), k-nearest Neighbors (k-NN), Support Vector Machine (SVM), and Neural Network (NN) based approaches can be used. Also, since CSI provides time series data, a Recurrent Neural Network (RNN) based architecture can be leveraged including but not limited to Long Short Term Memory (LSTM), and Gated Recurrent Unit (GRU). In another embodiment, in addition to mean, other statistical features are used for classification including but not limited to the median, standard deviation, variance, minimum, maximum, skewness, kurtosis, and histogram.

In another embodiment, a computer vision-based approach may be used for the classification. For example, S_{diff} values from a statistically significant population are fed through an encoder network, e.g., ResNet50, or VGG16, and the representation of the final layer is fed to a fully connected layer with two classes (fluid accumulation or not) for the classification. This may be useful for determining fluid accumulation in any organ and for other illnesses, e.g., cystic fibrosis, pneumonia, congestive heart failure, kidney failure, or liver cirrhosis.

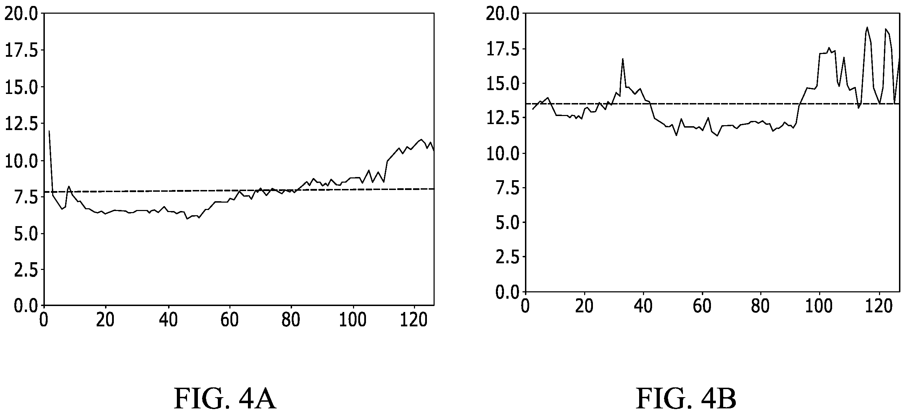

FIG. 4A illustrates a Wi-Fi fine-time measurement to determine fluid accumulation with a dry sponge. FIG. 4B illustrates a Wi-Fi fine-time measurement to determine fluid accumulation with a wet sponge. As shown, the data for the FTM with the dry sponge may below a certain value. However, with the FTM with the wet sponge, the data is shows more fluctuation and variability with higher average FTM distance measurement values (13 meters vs 7.5 meters). Thus, it can be determined utilizing FTM that when fluid is built up in a human organ (e.g., lung, fiver, etc.), as the FTM data being communicated between a transmitter and receiver may look more variable with higher average FTM distance measurement values.

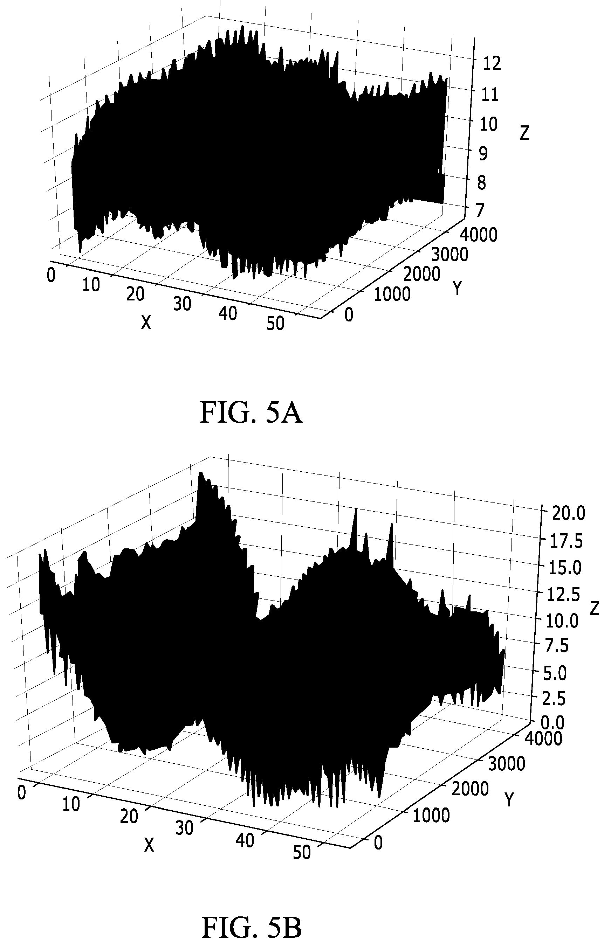

FIG. 5A illustrates a Wi-Fi channel state information to determine fluid accumulation with a dry sponge. FIG. 5B illustrates a Wi-Fi channel state information to determine fluid accumulation with a wet sponge. As shown, the data for the channel state information with the dry sponge may be more consistent and stable in CSI amplitude values. Thus, a threshold may be associated with consistency of data or values. However, with the channel state information with the wet sponge, the CSI amplitude has higher and lower values, with higher variation across different subcarriers indicating more fluctuations. Thus, it can be determined utilizing channel state information that when fluid is built up in a human organ (e.g., lung, fiver, etc.), as the CSI data being communicated between a transmitter and receiver may look more variable across the subcarriers with higher and lower amplitude values.

While exemplary embodiments are described above, it is not intended that these embodiments describe all possible forms encompassed by the claims. The words used in the specification are words of description rather than limitation, and it is understood that various changes can be made without departing from the spirit and scope of the disclosure. As previously described, the features of various embodiments can be combined to form further embodiments of the invention that may not be explicitly described or illustrated. While various embodiments could have been described as providing advantages or being preferred over other embodiments or prior art implementations with respect to one or more desired characteristics, those of ordinary skill in the art recognize that one or more features or characteristics can be compromised to achieve desired overall system attributes, which depend on the specific application and implementation. These attributes can include, but are not limited to cost, strength, durability, life cycle cost, marketability, appearance, packaging, size, serviceability, weight, manufacturability, ease of assembly, etc. As such, to the extent any embodiments are described as less desirable than other embodiments or prior art implementations with respect to one or more characteristics, these embodiments are not outside the scope of the disclosure and can be desirable for particular applications.

## Claims

1. A system, comprising:
a first and second transceiver configured to emit and receive a wireless signal; and
a controller in communication with the first and second transceiver, the controller configured to:
send instructions to the transceivers to initiate a Fine Time Measurement (FTM) on a wireless channel to a body part associated with a user during a healthy state of the user;
store an FTM measurement associated with the healthy state of the user;
send instructions to the transceiver to initiate the FTM on the wireless channel during an infected state of the user;
store an FTM measurement associated with the infected state of the user;
compare the FTM measurement associated with the healthy state of the user with the FTM measurement associated with the infected state of the user; and
in response to the comparison exceeding a threshold value, output a notification.

2. The system of claim 1, wherein the controller is further configured to perform channel hopping and send an FTM request for each Wi-Fi channel or a set of Wi-Fi channels by channel hopping.

3. The system of claim 1, wherein the notification includes a report indicating fluid accumulation.

4. The system of claim 1, wherein the threshold is variable in response to attributes associated with the user.

5. The system of claim 1, wherein the notification includes emitting a sound associated with a system speaker.

6. A system, comprising:
a first transceiver configured to emit a wireless signal;
a second transceiver configured to receive the wireless signal;
a controller in communication with the first transceiver and second transceiver, the controller configured to:
send instructions to the first transceiver to initiate a Fine Time Measurement (FTM) on a wireless channel to a body part associated with a user during a healthy state of the user;
store an FTM measurement associated with the healthy state of the user;
send instructions to the first transceiver to initiate the FTM on the wireless channel during an infected state of the user;
store an FTM measurement associated with the infected state of the user;
compare the FTM measurement associated with the healthy state of the user with the FTM measurement associated with the infected state of the user; and
in response to the comparison exceeding a threshold value, output a notification.

7. The system of claim 6, wherein the controller is further configured to perform channel hopping and send an FTM request for each Wi-Fi channel or a set of Wi-Fi channels by channel hopping.

8. The system of claim 6, wherein the notification includes a report indicating fluid accumulation.

9. The system of claim 6, wherein the threshold is variable in response to attributes associated with the user.

10. The system of claim 6, wherein the notification includes emitting a sound associated with a system speaker.

11. A system, comprising:
a transmitter configured to emit a wireless signal;
a receiver configured to receive the wireless signal and extract CSI data associated with the received signal;
a controller in communication with the transmitter and the receiver, the controller configured to:
send instructions to the transmitter to send a first set of packets on a wireless channel to a body part associated with a user during a healthy state of the user;
store a healthy CSI measurement associated with the healthy state of the user;
send instructions to the transceivers to initiate sending a second set of packets on the wireless channel during an infected state of the user;
store an infected CSI measurement associated with the infected state of the user;
compare the healthy CSI measurement associated with the healthy state of the user with the infected CSI measurement associated with the infected state of the user;
in response to the comparison exceeding a threshold value, output a notification.

12. The system of claim 11, wherein the notification includes a report indicating fluid accumulation.

13. The system of claim 11, wherein the threshold is variable in response to attributes associated with the user.

14. The system of claim 11, wherein the notification includes emitting a sound associated with a system speaker.

15. The system of claim 11, wherein the wireless signal with CSI indicates signal propagation from the transmitter to the receiver.

16. The system of claim 11, wherein the CSI measurement includes CSI amplitudes.

17. The system of claim 11, wherein the CSI measurement includes CSI phases.

18. The system of claim 11, wherein the plurality of packets include more than 10,000 packets.

19. The system of claim 11, wherein the comparison includes comparing amplitude values of subcarriers associated with CSI data.

20. The system of claim 11, wherein the threshold value is associated with consistency of CSI data.
